# EUROPEAN PATENT APPLICATION

(11) **EP 2 468 265 A2**
(43) Date of publication of application: **27.06.2012**
(21) Application number: 11156453.0
(22) Date of filing: 01.03.2011
(51) Int. Cl.: A61K 9/20, A61K 31/59, A23L 1/303, A61K 33/10

(54) **Homogeneous preparations containing vitamin D**

(30) Priority: 04.12.2010 SA 31088910
(71) Applicant: DEEF Pharmaceutical Industries Co., 51951 Al-Badaye (SA)
(72) Inventor: Mahmoud, Maged Fawzy Abdel Khalek, 51951 Al-Badaye (SA); Ebeed, Mohamed Ahmed Mohamed Kamal, 51951 Al-Badaye (SA)
(74) Representative: Rupp, Christian

(57) **Abstract**

The present invention provides a pharmaceutical and/or nutraceutical composition comprising vitamin D and a calcium salt for oral administration in the form of tablets or granules. A vitmin D dispersion comprising polyethylene glycol as a binder material, E320 and E321 as antioxidants and EDTA salt as sequestering agent in diluted volatile organic solvent is sprayed onto a powder bed of calcium salt and other excipients to provide a homogenous distribution of vitamin D in the pharmaceutical and/or nutraceutical composition comprising vitamin D and a calcium salt in order to enhance the performance of the formulation.

## Description

### Field of invention

The present invention relates to a pharmaceutical and/or nutraceutical composition comprising vitamin D and a calcium salt. The pharmaceutical and/or nutraceutical composition comprising vitamin D and the calcium salt is intended for oral administration and is in the form of tablets or granules. The present invention relates to a novel procedure for the manufacture of the pharmaceutical and/or nutraceutical composition comprising vitamin D and the calcium salt.

### Background of invention

A simultaneous use of vitamin D and calcium salts is ro utine i n pharmaceutical and/or nutraceutical compositions.

Vitamin D and calcium deficiency is related to the prevention and treatment of a number of disorders such as osteoporosis, bone defects and muscle defects. A supplement of vitamin D is usually accompanied with a calcium source. Recently it was identified that vitamin D deficiency may be a potential risk factor in cardiovascular diseases. The human requirements of vitamin D are increased during pr egnancy and after menopause. The supplement of vitamin D/calcium source dose is 200 IU/ 500 mg (equivalent to 0.0002/0.5 gm) and this raises a technical problem of how to obtain a uniform homogenous blend of the vitamin D/calcium source with an acceptable standard deviation value. Furthermore vitamin D is chemically unstable and may be degraded during storage of the supplement of vitamin D/calcium.

Efforts have been made to provide a supplement that combines vitamin D and calcium as for example described in US patent application No. 10/164,565. However in the US patent application No. 10/164,565 the vitamin D and calcium proportions in the resultant combination are 1:1 to 1.5:1 mg elemental calcium to IU vitamin D, these proportions are far from the desirable therapeutic combination ratio of 6:1 mg elemental calcium to IU vitamin D.

Vitamin D is available in many forms, such as pure crystals, a vitamin D in oil solution or as a diluted powder.

It is known that during mixing processes of the vitamin D in oil solution with other ingredients (such as pharmaceutical powders) to obtain a pharmaceutical blend depends on the concentrations of the vitamin D in the vitamin D in oil solution and the concentration of the other ingredients since the vitamin D quantity is usually minute. Such a minute quantity of the vitamin D results in high standard deviation for the distribution of the vitamin D within such a blend. In addition the stability of the vitamin D in the vitamin D in oil solution is affected by light, temperature, humidity, pH, oxidizing or reducing agents and the presence of metals.

The present invention aims to provide a simpler manufacture method than the methods described in the US patent No. 5,487,900, US. patent application No. 10/164,565 and US patent application No.12/148,987.

### Summary of the Invention

The present invention was contemplated in view of the prior art described above. An object of the present invention is to provide a practical method capable for the manufacture of a pharmaceutical and/or nutraceutical composition comprising vitamin D and a calcium salt. The method is easily implemented and reliable and leads to the formation of a stable pharmaceutical and/or nutraceutical composition comprising vitamin D and a calcium salt.

Most preparations containing vitamin D suffer with problems during preparation and storage. These problems can be a provision of a physically and chemically stable homogenous mixture.

The present inventors have found that preparation methods utilize vitamin D in the form of a diluted powder. The diluted powder has a relatively small particle size compared to a particle size of calcium salts to which the diluted powder is added during a mixing step. The differences in particle sizes leads to a physical instability that results from a lack of homogeneity which in turn leads to gradual segregation during handling in different stages of the manufacturing steps.

Also using diluted powder form of vitamin D may lead to high production cost.

The present invention has the advantage that the method for the manufacture of the vitamin D and calcium salt preparation which is a pharmaceutical and/or nutraceutical composition is simple. The method maintains the efficiency of physical and chemical properties and therapeutic value of the components of the preparation without using complex or expensive industrial tools and/or chemicals.

The present invention has superior advantages in that it does not contains or use any materials to adjust a pH and it also stabilizes the formulations by using antioxidants and sequestering agents. The present invention does not include a drying process and the present invention provides a simple preparation procedure which increases the productivity and decreases the cost.

The standard deviation of the homogeneity of vitamin D in the preparation is not more than 2%, which is less than what is known in the art.

### Detailed Description of Invention

For a complete understanding of the present invention and the advantages thereof, reference is made to the following detailed description of the invention

It should be appreciated that various embodiments of the present invention can be combined with other embodiments of the invention and are merely illustrative of the specific ways to make and use the invention and do not limit the scope of the invention when taken into consideration with the claims and the following detailed description.

The present invention is based on spraying a vitamin D dispersion on a calcium salt and/or other excipient to provide a homogenous mixture of the pharmaceutical and/or nutraceutical composition comprising vitamin D.

The vitamin D dispersion contains an adherent agent, an antioxidant, a chelating agent. The pharmaceutical and/or nutraceutical composition comprising vitamin D and the calcium salt has a standard deviation of less than 2% and high level of physical stability against particle segregation and high level of chemical stability.

Preparation steps of the vitamin D dispersion are performed as follows. A polyethylene glycol with a molecular weight of between approximately 1500 to 8000 and more preferably a molecular weight of between approximately between 3000 to 6000 which is used as adherent agent is dissolved in a diluted organic volatile solvent to form a mixture of the polyethylene glycol and diluted organic volatile solvent. To the mixture of the polyethylene glycol and diluted organic volatile solvent is added an antioxidant and vitamin D (oily form) which are then both dissolved. A chelating agent is then added. The chelating agent serves as metal extractor to enhance a chemical stability of vitamin D. This resulting mixture is the mixed to form the vitamin D dispersion

The diluted organic volatile solvent is one of methanol, ethanol and propanol. It is preferable that the diluted organic volatile solvent is ethanol due to its low toxicity. The dilution of the organic volatile solvent is performed using distilled and deionised water.

Following manufacture of the vitamin D dispersion, the vitamin D dispersion is sprayed on a calcium salt and/or other solid excipient using low shear or high shear.

The solid excipient can be any one of lactose, mannitol, microcrystalline cellulose, maize starch and/or xylitol or any combinations thereof.

The resulting pharmaceutical and/or nutraceutical composition comprising vitamin D and the calcium salt D can then be formed into granules by any method known in the art and also formed in tablets. Following the formation of granules or tablets an addition of pharmaceutically accepted additives can be performed according to the required final dosage form.

The pharmaceutical and/or nutraceutical composition comprising vitamin D and the calcium salt according to the present invention can be prepared according to the following method:
Dissolution of the polyethylene glycols in the diluted pharmaceutically accepted organic volatile solvent to form the mixture of the polyethylene glycol and diluted organic volatile solvent. Dissolve the antioxidants, such as but not limited to E320 and/or E321 in the mixture of the polyethylene glycol and diluted organic volatile solvent.

Disperse vitamin D (oily form) in the previous solution at room temperature.

Disperse the chelating agent to the previous mixture.

The resultant mixture is then mixed to form the vitamin D dispersion. The mixing can be performed by any of the following mixers: a turbine mixer, a cross beam mixer, a propeller mixer or a helical ribbon mixer.

The vitamin D dispersion is then sprayed on at least one of the following: a pharmaceutically accepted calcium salt, mannitol, colloidal silicone dioxide, xylitol, maize starch, lactose,

The spraying of the vitamin D dispersion on the calcium salt and/or other solid excipient is achieved using any one of the following granulators: high shear granulator or low shear granulator.

Following spraying of the vitamin D dispersion on the calcium salt and/or other solid excipient the composition is allowed to dry to form the pharmaceutical and/or nutraceutical composition comprising vitamin D and the calcium salt in a powder/granular form.

The pharmaceutical and/or nutraceutical composition comprising vitamin D and the calcium salt in the powder/granular form can be sifted using a sifter. A size of pores of the sifter are between 355 to 2000 micron.

Lubrication of the sifted pharmaceutical and/or nutraceutical composition comprising vitamin D and the calcium salt is achieved by using a pharmaceutically acceptable powder lubricator, such as, but not limited, magnesium stearate and sodium lauryl sulfate. The lubrication of the sifted pharmaceutical and/or nutraceutical composition comprising vitamin D and the calcium salt ensures a free flowing composition when in the powder form.

A compression and or packaging of the pharmaceutical and/or nutraceutical composition comprising vitamin D and the calcium salt can be done to achieve any of the following: uncoated tablets, film coated tablets, uncoated chewable tablets, dispersible tablets and free flowing granules.

The following non-limiting examples are herein used to illustrate the advantage of the present invention for the manufacture of the pharmaceutical and/or nutraceutical composition comprising vitamin D and the calcium salt.

### Examples

### Example 1

A pharmaceutical and/or nutraceutical composition comprising vitamin D and a calcium salt in the form of uncoated chewable tablets was manufactured according to the following procedure.
1. In a stainless steel container of a suitable size; polyethylene glycol 3350 (2.000 mg) was dissolved in 10 mg of ethanol 90% to form a mixture of polyethylene glycol and diluted organic volatile solvent.
2. The E320 (0.010 mg) and E321 (0.001 mg) was added and dissolved.
3. The vitamin D (oil form) - 200.0 I.U was dispersed at room temperature.
4. A chelating agent disodium edetate (0.001 mg) was dispersed using a propeller mixer for 10 minutes to form the vitamin D dispersion.
5. The vitamin D dispersion is sprayed on a mixture of granular mannitol (403.29 mg), colloidal silicone dioxide (8.00 mg), aspartame (7.500 mg) and granular calcium carbonate (1250 mg) using a high shear granulator at 50 rpm.
6. The sprayed powder is sifted through 1000 micron.
7. The powder is lubricated with magnesium stearate (8.00 mg).
8. Compress the powder into tablets using 20 kN compression force which leads to evaporation of ethanol due to the heat of the compression while the remaining water serves as a binder for the compressed tablets. It is noted that the maximum allowed ethanol residue in pharmaceutical and/or nutraceutical composition comprising vitamin D and the calcium salt should be less than 5000 ppm.

### Example 2

A pharmaceutical and/or nutraceutical composition comprising vitamin D and a calcium salt in the form of uncoated swallow able tablets was manufactured according to the following procedure.
1. In a stainless steel container of a suitable size; polyethylene glycol 6000 (2.000 mg) was dissolved in 10 mg of ethanol 90% to form a mixture of polyethylene glycol and diluted organic volatile solvent.
2. The E320 (0.010 mg) and E321 (0.001 mg) was added and dissolved.
3. The vitamin D (oil form) - 200.0 I.U was dispersed at room temperature.
4. A chelating agent disodium edetate (0.001 mg) was dispersed using a propeller mixer for 10 minutes to form the vitamin D dispersion.
5. The vitamin D dispersion is sprayed on a mixture of Lactose DC (403.29 mg), colloidal silicone dioxide (8.00 mg), aspartame (7.500 mg) and granular calcium carbonate (1250 mg) using a low shear granulator at 25 rpm.
6. The sprayed powder is sifted through 1000 micron.
7. The powder is lubricated with magnesium stearate (8.00 mg).
8. Compress the powder into tablets using 40 kN compression force which lead to evaporation of ethanol due to heat of compression while the remaining water serves as binder for the compressed tablets.

### Example 3

A pharmaceutical and/or nutraceutical composition comprising vitamin D and a calcium salt in the form of coated swallow able tablets was manufactured according to the following procedure.
1. In a stainless steel container of a suitable size; polyethylene glycol 4000 (2.000 mg) was dissolved in 10 mg of ethanol 90% to form a mixture of polyethylene glycol and diluted organic volatile solvent.
2. The E320 (0.010 mg) and E321 (0.001 mg) was added and dissolved.
3. The vitamin D (oil form) - 200.0 I.U was dispersed at room temperature.
4. A chelating agent disodium edetate (0.001 mg) was dispersed using a propeller mixer for 10 minutes to form the vitamin D dispersion.
5. The vitamin D dispersion is sprayed on a mixture Avicel PH 101 (403.29 mg), colloidal silicone dioxide (8.00 mg), aspartame (7.500 mg) and granular calcium carbonate (1250 mg) using a low shear granulator at 25 rpm.
6. The sprayed powder is sifted through 1000 micron.
7. The powder is lubricated with magnesium stearate (8.00 mg).
8. Compress the powder into tablets using 40 kN compression force which lead to evaporation of ethanol due to heat of compression while the remaining water serves as binder for the compressed tablets.
9. The tablets are coated with a coating dispersion made of aqueous dispersion of hydroxypropyl methylcellulose (40.0 mg), polysorbate 80 (2.0mg) and titanium dioxide (8.0mg).

### Example 4

A pharmaceutical and/or nutraceutical composition comprising vitamin D and a calcium salt in the form of dispersible tablets was manufactured according to the following procedure.
1. In a stainless steel container of a suitable size; polyethylene glycol 1500 (2.000 mg) was dissolved in 10 mg of ethanol 90% to form a mixture of polyethylene glycol and diluted organic volatile solvent.
2. The E320 (0.010 mg) and E321 (0.001 mg) was added and dissolved.
3. The vitamin D (oil form) - 200.0 I.U was dispersed at room temperature.
4. A chelating agent disodium edetate (0.001 mg) was dispersed using a propeller mixer for 10 minutes to form the vitamin D dispersion.
5. The vitamin D dispersion is sprayed on a mixture lactose (203.29 mg), colloidal silicone dioxide (8.00 mg), aspartame (7.50 mg) and granular calcium carbonate (1250 mg) using a low shear granulator at 25 rpm.
6. The sprayed powder is sifted through 1000 micron.
7. The powder is lubricated with sodium lauryl sulfate (8.00 mg).
8. Compress the powder into tablets using 10 kN compression force which lead to evaporation of ethanol due to heat of compression while the remaining water serves as binder for the compressed tablets.

### Example 5

A pharmaceutical and/or nutraceutical composition comprising vitamin D and a calcium salt in the form of dispersible granules was manufactured according to the following procedure.
1. In a stainless steel container of a suitable size; polyethylene glycol 1500 (2.000 mg) was dissolved in 10 mg of ethanol 90% to form a mixture of polyethylene glycol and diluted organic volatile solvent.
2. The E320 (0.010 mg) and E321 (0.001 mg) was added and dissolved.
3. The vitamin D (oil form) - 200.0 I.U was dispersed at room temperature.
4. A chelating agent disodium edetate (0.001 mg) was dispersed using a propeller mixer for 10 minutes to form the vitamin D dispersion.
5. The vitamin D dispersion is sprayed on a mixture of mannitol (411.29 mg), colloidal silicone dioxide (8.00 mg), aspartame (7.50 mg) and granular calcium carbonate (1250 mg) using a high shear granulator at 25 rpm.
6. The sprayed powder is sifted through 1000 micron.

The present invention provides a uniform homogenous blend of the vitamin D/calcium in the pharmaceutical and/or nutraceutical composition in proportions that are within the desirable therapeutic combination ratio of 6:1 mg elemental calcium to IU vitamin D. Furthermore the invention provides a composition in which the relative standard deviation of the vitamin D distribution is not more than 2% in the said composition.

The present invention avoids the need for separate drying steps as it uses volatile organic solvents. The present invention provides a simple preparation procedure which increases the productivity by avoiding steps of the prior art which would lead to loss of desired product.

Having thus described the present invention in detail and the advantages thereof, it is to be understood that the detailed description is not intended to limit the scope of the invention thereof.

What is desired to be protected by letters patent is set forth in the following claims.

## Claims

1. Pharmaceutical solid oral dosage form that is chemically and physically stable contains vitamin D and a calcium salt, in which the relative standard deviation of the vitamin D distribution is not more than 2%, the preparation method is based on spraying dispersion that contains oily form of vitamin D plus adherent agent, antioxidant and sequestering agent dispersed in diluted volatile organic solvent.

2. The solid oral dosage form according to claim 1 , wherein the calcium salt is one of the following salts or mixture of them; calcium carbonate, calcium lactate, calcium phosphate, calcium glubionate, calcium stearate and calcium sulphate.

3. The solid oral dosage form according to claim 1, wherein the dosage form is uncoated tablets, coated tablets, compressed uncoated chewable tablets, dispersible tablets or dispersible granules.

4. The solid oral dosage form according to claim 1, wherein the vitamin D solution contains adherent material which one of which is one of the following material or mixture of them; polyethylene glycols with molecular weight from about 1500 to 8000 more preferably from 3000 to 6000.

5. The solid oral dosage forms according to claim 1, wherein the vitamin D dispersion contains antioxidant which is one of the following material or mixture of them; E320 and E321.

6. The solid oral dosage form according to claim 1, wherein the vitamin D dispersion contains chelating (sequestering agent) which is one of the following material or mixture of them; edetic acid, dipotassium edetate, disodium edetate, trisodium edetate and edetate calcium disodium.

7. The solid oral dosage form according to claim 1, wherein the vitamin D solution prepared using diluted organic volatile solvent) which is one of the following material or mixture of them; methanol, ethanol and propanol.
